# EUROPEAN PATENT APPLICATION

(11) **EP 2 055 305 A1**
(43) Date of publication of application: **06.05.2009**
(21) Application number: 07792383.7
(22) Date of filing: 10.08.2007
(51) Int. Cl.: A61K 31/5575, A61K 9/06, A61K 9/08, A61K 47/40, A61P 17/00, A61P 37/08

(54) **EXTERNAL PREPARATION COMPRISING PROSTAGLANDIN DERIVATIVE**

(30) Priority: 11.08.2006 JP 2006220720
(71) Applicant: Taisho Pharmaceutical Co. Ltd., Tokyo 170-8633 (JP)
(72) Inventor: SATO, Takayuki, Tokyo 170-8633 (JP); SATO, Yoshie, Morioka-shi Iwate 020-0875 (JP)
(74) Representative: HOFFMANN EITLE
(86) International application number: PCT/JP2007/065738
(87) International publication number: WO 2008/018592

(57) **Abstract**

Provided is an external preparation comprising a complex containing: any one of a prostaglandin derivative, a pharmaceutically acceptable salt thereof and a hydrate thereof; and cyclodextrin, the prostaglandin derivative being represented by the following formula (I) : 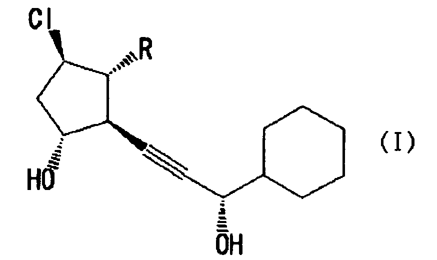 (where R represents a group represented by the formula: -(CH₂)₄-S-CH₂-CO₂H, - (CH₂)₄-S-CH₂-CO₂CH₃, - (CH₂)₄-C≡C-CO₂H, -CH₂-S- (CH₂)₂-S-CH₂-CO₂H, or -CH₂-S-(CH₂)₄-CO₂H), so that storage stability and content uniformity of the compound of the formula (I) are ensured.

## Description

### Technical Field

The present invention relates to an external preparation containing a prostaglandin derivative and having excellent storage stability and content uniformity of the prostaglandin derivative.

### Background of the Invention

Conventionally, steroid drugs, immunosuppressive drugs and the like have been used as therapeutic agents for atopic dermatitis. However, these drugs are still insufficiently effective, and also not sufficiently satisfactory from the view point of side effects.

In this regard, drugs comprising prostaglandin derivatives which exert an excellent therapeutic effect on pruritic symptoms such as itching caused by atopic dermatitis are provided. That is, it is a prophylactic agent or a therapeutic agent for pruritic symptoms comprising a prostaglandin derivative, a pharmaceutically acceptable salt thereof, or a hydrate thereof as an active ingredient, the prostaglandin derivative being represented by the following formula (I):

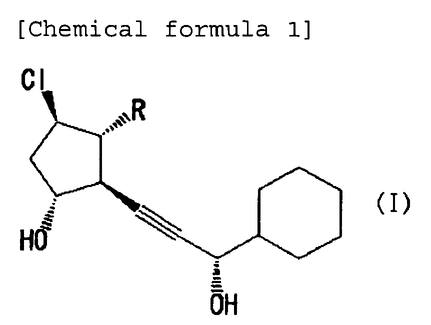

(where R represents a group represented by the formula: -(CH₂)₄-S-CH₂-CO₂H, (CH₂)₄-S-CH₂-CO₂CH₃, - -(CH₂)₄-C≡C-CO₂H, -CH₂-S- (CH₂)₂-S-CH₂-CO₂H, or -CH₂-S- (CH₂)₄-CO₂H) (refer to Patent Document 1).
Patent Document 1: International Patent Publication No. WO2004/014394

### Disclosure of the Invention

### Problem to be Solved by the Invention

However, the compound represented by the formula (I) (hereinafter arbitrarily referred to as "compound of formula I") has poor storage stability in a dissolved or dispersed state, and this constitutes obstacles to ensuring storage stability when the compound of formula I is used as an external preparation. Moreover, since the compound of formula I is rapidly decomposed at a temperature which exceeds its melting point, it is difficult to prepare an external preparation using a base which softens above the melting point of the compound of formula I.

Consequently, an object of the present invention is to provide an external preparation containing the compound of formula I and having excellent storage stability and content uniformity.

### Means for Solving the Problem

The present inventors have earnestly studied in order to resolve the problems described above. As a result, the inventors have found an external preparation which has excellent storage stability and content uniformity of the compound of formula I; the external preparation being made by dissolving or dispersing, into a base for external preparations, a powder obtained by grinding a block prepared by freeze-drying a solution which is obtained by dissolving the compound of formula I and a cyclodextrin into water, or a powder obtained by mixing and grinding the compound of formula I and a cyclodextrin; or the external preparation being made by dissolving the compound of formula I into an aqueous solution containing a cyclodextrin dissolved therein. Moreover, the inventors have also found that the powder containing the compound of formula I is stable above the melting point of the compound of formula I, and the powder can be prepared as an external preparation such as an ointment preparation even at a certain level of high temperature.

An aspect according to the present invention based on the above-described findings is an external preparation comprising a complex containing any one of a prostaglandin derivative, a pharmaceutically acceptable salt thereof and a hydrate thereof; and a cyclodextrin, the prostaglandin derivative being represented by the following formula (I): 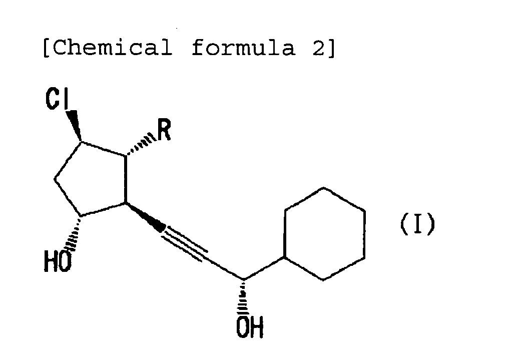 (where R represents a group represented by the formula: - (CH₂)₄-S-CH₂-CO₂H, -(CH₂)₄-S-CH₂-CO₂CH₃, - (CH₂)₄-C≡C-CO₂H, -CH₂-S-(CH₂)₂-S-CH₂-CO₂H, or -CH₂-S-(CH₂)₄-CO₂H).

### Effect of the Invention

According to the present invention, it possible to provide an external preparation containing the compound of formula I and having excellent storage stability and content uniformity, even when the concentration of the compound is low.

### Detailed Description of the Preferred Embodiments

Any compounds of formula I in the present invention have an excellent therapeutic effect for pruritic symptoms. For example, these compounds can be used for an antipruritic agent to itching caused by atopic dermatitis. A method for producing this antipruritic agent is described in WO2004/014394 (refer to Patent Document 1).

Among the compounds of formula I, a preferable compound in the present invention is a prostaglandin derivative represented by the following formula (II), a pharmaceutically acceptable salt thereof or a hydrate thereof (hereinafter arbitrarily referred to as "compound of formula II"), from a viewpoint of storage stability, content uniformity thereof, and the like.

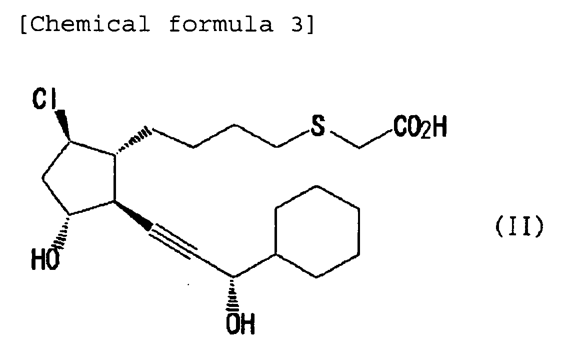

The melting point of the compound of formula II is approximately 55°C and the solubility in water at 25°C is approximately 0.06 mg/mL.

A content (a formulation amount) of the compound of formula I in an external preparation is 0.00001 to 1% by mass, and preferably 0.00001 to 0.1% by mass. For the compound of formula I exerting sufficient efficacy as an antipruritic agent even in an extremely small amount, it is very significant to ensure storage stability and content uniformity at a low concentration of especially 0.01% by mass or less, and hence a content of such a compound of formula I in an external preparation is more preferably 0.00001 to 0.01% by mass, and further preferably 0.00001 to 0.001% by mass.

The "cyclodextrin" is not limited as long as the cyclodextrin can be formulated in an external preparation. For example, α-cyclodextrin, β-cyclodextrin, γ-cyclodextrin and derivatives thereof can be included. These may be used singly or in combination of two or more.

A content (a formulation amount) of cyclodextrin depends on types of external preparations. For example, for external preparations containing an active ingredient dispersed therein, such as ointment preparation, a content of cyclodextrin is not substantially limited, and the acceptable range of the content is approximately 50% by mass in a whole external preparation merely from a viewpoint of sense of use or other factors. Thus, a content (a formulation amount) of cyclodextrin in external preparations containing an active ingredient dispersed therein, such as an ointment preparation, is 35 to 216400 moles, and more preferably 35 to 21640 moles for α-cyclodextrin per mole of the compound of formula I. For β-cyclodextrin, the content is 3 to 185500 moles, and more preferably 37 to 18550 moles. For γ-cyclodextrin, the content is 6 to 162300 moles, and more preferably 6 to 16230 moles. For hydroxypropyl-β-cyclodextrin, which is a derivative of β-cyclodextrin, the content is 7 to 139700 moles, and more preferably 7 to 13970 moles. In contrast, for external preparations containing an active ingredient dissolved therein such as a lotion preparation, a content of cyclodextrin is limited to narrower range than that of external preparations containing an active ingredient dispersed therein, such as an ointment preparation, because of limitation caused by solubility of cyclodextrin into water. Therefore, a content (a formulation amount) of cyclodextrin in external preparations containing an active ingredient dissolved therein, such as a lotion preparation, is 35 to 60600 moles, and more preferably 43 to 43300 moles for α-cyclodextrin per mole of the compound of formula I. For β-cyclodextrin, the content is 37 to 7400 moles, and more preferably 37 to 5560 moles. For γ-cyclodextrin, the content is 6 to 74600 moles, and more preferably 6 to 48700 moles. For hydroxypropyl-β-cyclodextrin, which is a derivative of β-cyclodextrin, the content is 7 to 61400 moles, and more preferably 7 to 41900 moles.

The term "complex" in the present invention means one being in a following coexistence state of the compound of formula I and the cyclodextrin. That is, the "complex" means one being in the coexistence states in which change in properties such as solubility can be observed in comparison with the original compound of formula I, for example, a powder obtained by grinding a block prepared by freeze-drying a solution which is obtained by dissolving the compound of formula I and the cyclodextrin into water, or a powder obtained by mixing and grinding the compound of formula I and the cyclodextrin. It is speculated that such a state occurs when the compound of formula I is merely dissolved into an aqueous solution containing the cyclodextrin dissolved therein, and the form thereof is a powder, solution or the like.

Examples of "external preparations" include a lotion preparation, an aerosol preparation, an ointment preparation, a cream preparation, a gel preparation and a patch application. However, from a viewpoint of ensuring storage stability and content uniformity in a low concentration of the compound of formula I, an ointment preparation is preferable as an external preparation containing an active ingredient dispersed therein and a lotion preparation is preferable as an external preparation containing an active ingredient dissolved therein.

Here, in carriers formulated in an external preparation, an oleaginous base, an aqueous base, an emulsion base and the like can be included.

Examples of "oleaginous bases" include a petrolatum such as a white petrolatum; a paraffin, a hardened oil and a wax. It is very significant to use the present invention when an oleaginous base, which needs to be softened by being heated at a temperature equal to the melting point of the compound of formula I or higher during formulation of external preparations such as an ointment preparation, is used.

In "aqueous bases", polyethylene glycol, glycerin, 1,3-butylene glycol and the like can be included.

The external preparation of the present invention is produced, for example, by the following method.

A solvent or an additive is added to the compound of formula I and the cyclodextrin to prepare a solution. Then, the solution is freeze-dried to prepare a block, and the block is grinded to obtain a powder; alternatively, mixing and grinding is performed without freeze-drying to obtain a powder. The obtained powder is added to a solvent or an additive, and uniformly dissolved or dispersed. Thus, an external preparation such as an ointment preparation can be obtained.

Here, depending on characteristics of the additive or the external preparation, it is needed to produce the external preparation at a temperature of the melting point of the compound of formula I or higher in some cases. However, since the above-described powder is stable even at a temperature higher than the melting point of the compound of formula I, an external preparation containing the compound of formula I can be easily produced.

Particularly, when an ointment preparation is produced by dispersing a powder containing the compound of formula II and α-cyclodextrin into an oleaginous base, it is possible to produce an ointment preparation having excellent storage stability and mixing uniformity of the compound of formula II contained in an extremely small content, even if the powder containing the compound of formula II and α-cyclodextrin is added and mixed to the molten oleaginous base, at a temperature higher than the melting point of the compound of formula II.

Moreover, an external preparation containing the compound of formula I such as a lotion preparation also can be obtained by dissolving the cyclodextrin into a solvent such as water and then dissolving the compound of formula I into the solution.

To the external preparation of the present invention, another known additive, for example, an antioxidant such as butylhydroxyanisole or dibutylhydroxytoluene, a stabilizer such as EDTA-2Na, and another additive can be optionally added, as long as effects of the present invention are not impaired.

### Examples

The present invention is described in great detail below by exemplifying Examples, Comparative Examples and Test Examples.

Note that Triomaster A-04 manufactured by Kyowa Vacuum Engineering, Ltd. was used as a freeze-dryer. Fine Impact Mill 100UPZ-II manufactured by Hosokawa Micron Corporation was used as a hammer mill. A ball mill manufactured by Fritsch GmbH was used. PVQ-3T manufactured by MIZUHO Industrial CO., LTD. was used as a vacuum emulsification-agitation device.

### Example 1

2 g of β-cyclodextrin was added to and dissolved into 98 g of a liquid obtained by diluting a disodium hydrogen phosphate (Na₂HPO₃)·citric acid buffer solution (pH 6.0) fivefold with purified water. Then, 20 mg of the compound of formula II was dissolved into the solution to obtain a lotion preparation containing the compound of formula II at a content of 0.02% by mass.

### Example 2

After dissolving 2 g of α-cyclodextrin into 98 g of purified water, 20 mg of the compound of formula II was dissolved into the solution to obtain a lotion preparation containing the compound of formula II at a content of 0.02% by mass.

### Example 3

After dissolving 5 g of α-cyclodextrin into 95 g of purified water, 20 mg of the compound of formula II was dissolved into the solution to obtain a lotion preparation containing the compound of formula II at a content of 0.02% by mass.

### Example 4

After dissolving 5 g of γ-cyclodextrin into 95 g of purified water, 10 mg of the compound of formula II was dissolved into the solution to obtain a lotion preparation containing the compound of formula II at a content of 0.01% by mass.

### Example 5

0.5 g of the compound of formula II and 100 g of α-cyclodextrin was dissolved into 1000 mL of purified water, and the solution was freeze-dried with the freeze-dryer to obtain a block. The block was ground with the hammer mill to obtain a powder containing the compound of formula II and α-cyclodextrin. 20 mg of the powder containing the compound of formula II and α-cyclodextrin was added to and mixed with 25 g of melted white petrolatum which was heated to approximately 65°C. After sieving the mixture with a sieve having a mesh size of 150 µm, the mixture was mixed with 75 g of white petrolatum heated to approximately 65°C and uniformly dispersed by use of the vacuum emulsification-agitation device. The obtained mixture was cooled and solidified with agitation to obtain an ointment preparation containing the compound of formula II at a content of 0.0001% by mass.

### Example 6

1 mg of the compound of formula II and 9 mg of β-cyclodextrin was mixed and ground with the ball mill to obtain a powder containing the compound of formula II and β-cyclodextrin. 10 mg of the powder containing the compound of formula II and β-cyclodextrin was added to and mixed with 10 g of melted white petrolatum which was heated to approximately 50°C. After sieving, the mixture was mixed with 90 g of white petrolatum heated to approximately 50°C and uniformly dispersed by use of the vacuum emulsification-agitation device. The obtained mixture was cooled and solidified with agitation to obtain an ointment preparation containing the compound of formula II at a content of 0.001% by mass.

### Example 7

200 mg of the powder containing the compound of formula II and α-cyclodextrin prepared in Example 5 was added to and mixed with 25 g of melted white petrolatum which was heated to approximately 65°C. After sieving the mixture with a sieve having a mesh size of 150 µm, the mixture was mixed with 75 g of white petrolatum heated to approximately 65°C and uniformly dispersed by use of the vacuum emulsification-agitation device. The obtained mixture was cooled and solidified with agitation to obtain an ointment preparation containing the compound of formula II at a content of 0.001% by mass.

### Example 8

0.2 g of the compound of formula II and 100 g of α-cyclodextrin was dissolved into 1000 mL of purified water, and the solution was freeze-dried with the freeze-dryer to obtain a block. The block was ground with the hammer mill to obtain a powder containing the compound of formula II and α-cyclodextrin. 50 mg of the powder containing the compound of formula II and α-cyclodextrin was added to and mixed with 25 g of melted white petrolatum which was heated to approximately 65°C. After sieving the mixture with a sieve having a mesh size of 150 µm, the mixture was mixed with 75 g of white petrolatum heated to approximately 65°C and uniformly dispersed by use of the vacuum emulsification-agitation device. The obtained mixture was cooled and solidified with agitation to obtain an ointment preparation containing the compound of formula II at a content of 0.0001% by mass.

### Example 9

0.1 g of the compound of formula II and 100 g of α-cyclodextrin was dissolved into 1000 mL of purified water, and the solution was freeze-dried with the freeze-dryer to obtain a block. The block was ground with the hammer mill to obtain a powder containing the compound of formula II and α-cyclodextrin. 100 mg of the powder containing the compound of formula II and α-cyclodextrin was added to and mixed with 25 g of melted white petrolatum which was heated to approximately 65°C. After sieving the mixture with a sieve having a mesh size of 150 µm, the mixture was mixed with 75 g of white petrolatum heated to approximately 65°C and uniformly dispersed by use of the vacuum emulsification-agitation device. The obtained mixture was cooled and solidified with agitation to obtain an ointment preparation containing the compound of formula II at a content of 0.0001% by mass.

### Example 10

0.05 g of the compound of formula II and 100 g of α-cyclodextrin was dissolved into 1000 mL of purified water, and the solution was freeze-dried with the freeze-dryer to obtain a block. The block was ground with the hammer mill to obtain a powder containing the compound of formula II and α-cyclodextrin. 200 mg of the powder containing the compound of formula II and α-cyclodextrin was added to and mixed with 25 g of melted white petrolatum which was heated to approximately 65°C. After sieving the mixture with a sieve having a mesh size of 150 µm, the mixture was mixed with 75 g of white petrolatum heated to approximately 65°C and uniformly dispersed by use of the vacuum emulsification-agitation device. The obtained mixture was cooled and solidified with agitation to obtain an ointment preparation containing the compound of formula II at a content of 0.0001% by mass.

### Example 11

500 mg of the powder containing the compound of formula II and α-cyclodextrin prepared in Example 8 was added to and mixed with 25 g of melted white petrolatum which was heated to approximately 65°C. After sieving the mixture with a sieve having a mesh size of 150 µm, the mixture was mixed with 75 g of white petrolatum heated to approximately 65°C and uniformly dispersed by use of the vacuum emulsification-agitation device. The obtained mixture was cooled and solidified with agitation to obtain an ointment preparation containing the compound of formula II at a content of 0.001% by mass.

### Example 12

1000 mg of the powder containing the compound of formula II and α-cyclodextrin prepared in Example 9 was added to and mixed with 25 g of melted white petrolatum which was heated to approximately 65°C. After sieving the mixture with a sieve having a mesh size of 150 µm, the mixture was mixed with 75 g of white petrolatum heated to approximately 65°C and uniformly dispersed by use of the vacuum emulsification-agitation device. The obtained mixture was cooled and solidified with agitation to obtain an ointment preparation containing the compound of formula II at a content of 0.001% by mass.

### Example 13

1 g of the compound of formula II and 100 g of α-cyclodextrin was dissolved into 1000 mL of purified water, and the solution was freeze-dried with the freeze-dryer to obtain a block. The block was ground with the hammer mill to obtain a powder containing the compound of formula II and α-cyclodextrin.

### Comparative Example 1

5 mg of the compound of formula II was dissolved into 100 mL of physiological saline solution to obtain a lotion preparation containing the compound of formula II at a content of 0.005% by mass.

### Comparative Example 2

1 g of dextran, as a solubilizer, was added to and dissolved into 99 g of a liquid obtained by diluting a disodium hydrogen phosphate (Na₂HPO₃)·citric acid buffer solution (pH 6.0) fivefold with purified water. Then, 20 mg of the compound of formula II was dissolved into the solution to obtain a lotion preparation containing the compound of formula II at a content of 0.02% by mass.

### Comparative Example 3

0.1 mg of the compound of formula II was added to and mixed with 10 g of white petrolatum. The mixture was mixed with 90 g of white petrolatum heated to approximately 50°C and uniformly dispersed by use of the vacuum emulsification-agitation device. The obtained mixture was cooled and solidified with agitation to obtain an ointment preparation containing the compound of formula II at a content of 0.0001% by mass.

### Comparative Example 4

0.1 mg of the compound of formula II was added to and mixed with 5 g of light liquid paraffin to be uniformly dispersed. The dispersion liquid containing the compound of formula II was added to and mixed with 95 g of white petrolatum heated to approximately 50°C and was uniformly dispersed by use of the vacuum emulsification-agitation device. The obtained mixture was cooled and solidified with agitation to obtain an ointment preparation containing the compound of formula II at a content of 0.0001% by mass.

### Comparative Example 5

1 mg of the compound of formula II was added to and mixed with 5 g of light liquid paraffin to be uniformly dispersed. The dispersion liquid containing the compound of formula II was added to and mixed with 95 g of white petrolatum heated to approximately 50°C and was uniformly dispersed by use of the vacuum emulsification-agitation device. The obtained mixture was cooled and solidified with agitation to obtain an ointment preparation containing the compound of formula II at a content of 0.001% by mass.

### Comparative Example 6

1 mg of the compound of formula II was added to and mixed with 10 g of white petrolatum. The mixture was mixed with 90 g of white petrolatum heated to approximately 50°C and uniformly dispersed by use of the vacuum emulsification-agitation device. The obtained mixture was cooled and solidified with agitation to obtain an ointment preparation containing the compound of formula II at a content of 0.001% by mass.

### Comparative Example 7

1 mg of the compound of formula II was added to and mixed with 10 g of melted white petrolatum which was heated to approximately 80°C, and then completely melted. The mixture was mixed with 90 g of white petrolatum heated to approximately 60°C and uniformly dispersed by use of the vacuum emulsification-agitation device. The obtained mixture was cooled and solidified with agitation to obtain an ointment preparation containing the compound of formula II at a content of 0.001% by mass.

### Comparative Example 8

10 mg of dibutylhydroxytoluene was added to and mixed with 10 g of melted white petrolatum which was heated to approximately 80°C. 1 mg of the compound of formula II was further added to and mixed with the mixture, and then completely melted. The mixture was mixed with 90 g of white petrolatum heated to approximately 60°C and uniformly dispersed by use of the vacuum emulsification-agitation device. The obtained mixture was cooled and solidified with agitation to obtain an ointment preparation containing the compound of formula II at a content of 0.001% by mass.

### Test Example 1

In order to determine the storage stability of the compound of formula II in the lotion preparations, content (%) to the initial state of the compound of formula II in the lotion preparations was measured under various storage temperatures. The results are listed in Table 1.

**[Table 1]**

| | | Comp. Ex. 1 | Comp. Ex. 2 | Ex. 1 | Ex. 2 | Ex. 3 | Ex. 4 |
|---|---|---|---|---|---|---|---|
| Concentration of compound of formula II | | 0.005% | 0.02% | 0.02% | 0.02% | 0.02% | 0.01% |
| Content to initial state (%) | Initial | 100.0 | 100.0 | 100.0 | 100.0 | 100.0 | 100.0 |
| | 50°C, 1 week | 83.7 | - | - | - | - | - |
| | 50ºC, 2 weeks | 71.9 | 78.9 | 88.6 | 85.8 | 90.2 | 96.9 |
| | 50°C, 4 weeks (1 month) | - | 60.4 | 72.8 | 74.7 | 80.1 | 91.8 |
| | 40°C, 1 month (4 weeks) | - | 90.0 | 90.6 | 93.1 | 93.4 | 92.2 |

Since the compound of formula II has low solubility in water, the lotion preparation of Comparative Example 1 had such a low concentration that the content of the compound of formula II was 0.005%. The content after two-week storage at 50°C was 71.9%. In Comparative Example 2, dextran was added in order to increase solubility of the compound of formula II, and the examination was performed at a concentration of 0.02%. The content after two-week storage at 50°C was 78.9%.

On the other hand, in Examples 1 to 3, in which a cyclodextrin was added, the content in each 0.02% lotion preparation after two-week storage at 50°C was a high value of 85% or more.

Moreover, in Example 4, in which γ-cyclodextrin was added, examination was performed on the 0.01% lotion preparation because solubilization effect for the compound of formula II was insufficient. However, the content after two-week storage at 50°C was also a high value of 96.9%. Thus, it was found out that storage stability of the compound of formula II was improved by formulating a cyclodextrin to the solutions thereof.

### Test Example 2

In order to determine the storage stability of the compound of formula II in the ointment preparations, content (%) to the initial state of the compound of formula II in the ointment preparations were measured under various storage temperatures. The results are listed in Table 2.

**[Table 2]**

| | | Examination at concentration of compound of formula II of 0.0001% | | | Examination at concentration of compound of formula II of 0.001% | | |
|---|---|---|---|---|---|---|---|
| | | Comp. Ex. 3 | Comp. Ex. 4 | Ex. 5 | Comp. Ex. 5 | Ex. 6 | Ex. 7 |
| Content to initial state (%) | Initial | 100.0 | 100.0 | 100.0 | 100.0 | 100.0 | 100.0 |
| | 50°C, 2 weeks | 80.8 | 66.5 | 85.1 | 89.3 | 92.0 | 96.6 |
| | 50°C, 4 weeks | 67.5 | 61.7 | 77.8 | 79.9 | 89.4 | 96.8 |
| | 40°C, 1 month | - | 82.2 | 90.5 | - | - | 95.6 |
| | 40°C, 3 months | 58.7 | - | 74.7 | 75.5 | 80.8 | 91.8 |
| | 25°C, 3 months | 82.1 | - | 98.4 | 92.7 | - | 98.9 |

When the ointment preparations containing the compound of formula II in the same concentration were stored under the same conditions, contents (%) to the initial state showed high values in the ointment preparations in Examples 5, 6 and 7, in which cyclodextrin was formulated.

Therefore, for the ointment preparation, it was found out that the storage stability of the compound of formula II was improved by formulating cyclodextrin to the ointment preparation.

### Test Example 3

In order to examine the storage stability of the compound of formula II in the ointment preparations in greater detail, contents (%) to the initial state of the compound of formula II in the ointment preparations were measured under various storage temperatures, for the ointment preparations in Example 5, 8, 9 and 10, in which formulation ratios of α-cyclodextrin were varied. The results are listed in Table 3.

**[Table 3]**

| | | Examination at concentration of compound of formula II of 0.0001% | | | | |
|---|---|---|---|---|---|---|
| | | Comp. Ex. 3 | Ex.5 | Ex.8 | Ex. 9 | Ex.10 |
| Mass ratio of cyclodextrin to compound of formula II | | None | 1:200 | 1:500 | 1:1000 | 1:2000 |
| Molar ratio of cyclodextrin to compound of formula II | | None | 1:87 | 1:216 | 1:433 | 1:865 |
| Content to initial state (%) | Initial | 100.0 | 100.0 | 100.0 | 100.0 | 100.0 |
| | 50°C, 2 weeks | 80.8 | 85.1 | - | 96.1 | 92.4 |
| | 50°C, 4 weeks | 67.5 | 77.8 | 95.9 | 93.9 | 88.1 |
| | 3 month | 58.7 | 74.7 | 95.0 | 92.5 | - |
| | 25°C, 3 months | 82.1 | 98.4 | 99.3 | 96.2 | - |

When the contents under the same storage condition were compared, all the ointment preparations in Examples 5, 8, 9 and 10 showed higher content than the ointment preparation in Comparative Example 3. It was found out that the storage stability was improved over a wide range of a molar ratio of α-cyclodextrin of 87 to 865 per mole of the compound of formula II.

### Test Example 4

In order to compare content uniformity of the compound of formula II in the ointment preparations, samples were taken from 5 to 6 locations in each ointment preparation. Then, an average and a relative standard deviation (RSD) of the content of the compound of formula II was determined. The results are listed in Table 4.

**[Table 4]**

| | | Examination at concentration of compound of formula II of 0.001% | | | | Examination at concentration of compound of formula II of 0.0001 % | | | |
|---|---|---|---|---|---|---|---|---|---|
| | | Comp. Ex. 6 | Ex.7 | Ex. 11 | Ex.12 | Comp. Ex. 3 | Ex.5 | Ex.8 | Ex. 9 |
| Molar ratio of cyclodextrin | | None | 1:87 | 1:216 | 1:433 | None | 1:87 | 1:216 | 1:433 |
| Content (%) | Average | 97.4 | 101.2 | 103.8 | 104.4 | 102.5 | 94.7 | 98.7 | 107.7 |
| | RSD | 4.5 | 1.3 | 0.9 | 0.2 | 5.7 | 2.6 | 1.9 | 0.7 |

All the ointment preparations in Comparative Examples, in which the contents of the compound of formula II were 0.001% and 0.0001% showed high relative standard deviations exceeding 4%.

On the other hand, all the ointment preparations in Examples containing cyclodextrin showed relative standard deviations of 3% or less, and thus content uniformity was improved.

### Test Example 5

In order to verify the storage stability of the compound of formula II during the production process of the ointment preparations, at a temperature exceeding approximately 55°C, which is the melting point of the compound of formula II, an averages and relative standard deviations (RSD) of the content of the compound of formula II in all the ointment preparations, which is produced by dispersing the compound of formula II in a base, in Comparative Examples 7 and 8 and Examples 7 and 11 were compared. The results are listed in Table 5.

**[Table 5]**

| | | Examination at concentration of compound of formula II of 0.001 % | | | |
|---|---|---|---|---|---|
| | | Comp. Ex. 7 | Comp. Ex. 8 | Ex. 7 | Ex.11 |
| Content (%) | Average | 35.0 | 26.7 | 101.2 | 103.8 |
| | RSD | 23.1 | 14.7 | 1.3 | 0.9 |

In Comparative Example 7, in which only the compound of formula II was dispersed, the compound of formula II decomposed during the production, so that the content was significantly decreased. This decomposition could not be prevented even in Comparative Example 8, in which dibutylhydroxytoluene, which is an antioxidant, was added in advance.

On the other hand, in Examples 7 and 11, in each of which the powder obtained by grinding the freeze-dried mixture of the compound of formula II and α-cyclodextrin was dispersed, decrease in the content was not observed, that is, the ointment preparations were stable.

### Test Example 6

In order to further examine the stabilization of the compound of formula II by α-cyclodextrin, which was observed in Test Example 5, 5 mg of the powder in Example 13 was mixed with 5 g of white petrolatum and the mixture was heated to 70°C exceeding the melting point of the compound of formula II, and change in content (%) to the initial state of the compound of formula II was measured. The results are listed in Table 6.

**[Table 6]**

| Heating time of powder in white petrolatum | Content to initial state (%) of compound of formula II |
|---|---|
| Initial | 100.0 |
| 70°C, 30 min | 98.6 |
| 70°C, 60 min | 100.0 |
| 70°C, 120 min | 99.0 |

As for the powder obtained by grinding the freeze-dried mixture of the compound of formula II and α-cyclodextrin, decrease in the content was not observed up to 120 min even in a heated state at 70°C where a production of ointment preparations was assumed, that is, the compound of formula II was stable.

### Test Example 7

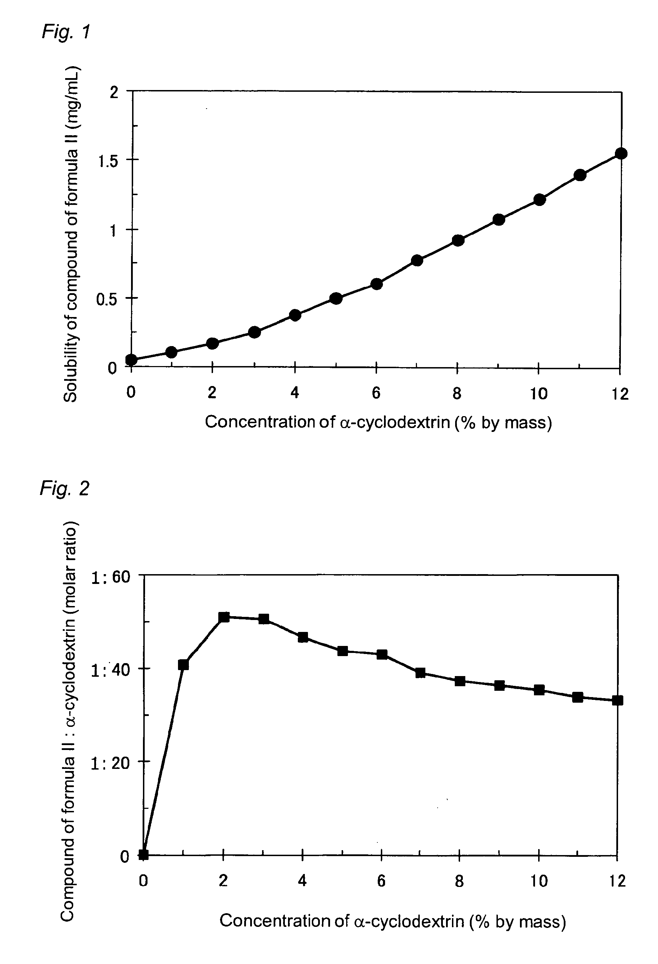

A complex containing the compound of formula II and the cyclodextrin can be obtained by grinding a block prepared by freeze-drying a solution which is obtained by dissolving the both into water. In order to examine the minimum content (formulation amount) of cyclodextrin required to obtain the complex, solubility of the compound of formula II in cyclodextrin aqueous solutions which have different concentrations of and different types of cyclodextrin was measured. That is, cyclodextrin aqueous solutions of various concentrations were prepared by dissolving α-cyclodextrin, β-cyclodextrin, γ-cyclodextrin or hydroxypropyl-β-cyclodextrin into water. After each solution was poured into a test tube, the compound of formula II was added excessively. The test tube was shaken in a water bath at 25°C for 24 hours, and a concentration of the dissolved compound of formula II was quantified with high-performance liquid chromatography (Equipment: LC10VP series manufactured by SHIMADZU CORPORATION, Column: Develosil ODS-HG-5 manufactured by Nomura Chemical Co., Ltd., Inner diameter: 4.6 mm, Length: 15 cm, Column temperature: 40°C, Mobile phase: mixed solution of water/ acetonitrile/ phosphoric acid (550:450:1), Flow rate: 1 mL/min, Detection wavelength: 200 nm). With this, solubility of the compound of formula II and molar ratio of the compound of formula II and each type of cyclodextrin were calculated. The results are illustrated in Figure 1, Figure 2 and listed in Table 7.

Note that, for calculation of the molar ratios, molecular weights of the compound of formula II, α-cyclodextrin, β-cyclodextrin, γ-cyclodextrin and hydroxypropyl-β-cyclodextrin were set to 420.99, 972.84, 1134.98, 1297.12 and 1506.69, respectively.

**[Table 7]**

| | | | | |
|---|---|---|---|---|
| Concentration of α-cyclodextrin (% by mass) | 1 | 2 | 5 | 10 |
| Solubility of compound of formula II (mg/mL) | 0.11 | 0.17 | 0.5 | 1.22 |
| Compound of formula II: cyclodextrin (molar ratio) | 1:41 | 1:51 | 1:44 | 1:35 |
| | | | | |
| Concentration of β-cyclodextrin (% by mass) | 1 | 2 | | |
| Solubility of compound of formula II (mg/mL) | <0.10 | 0.20* | | |
| Compound of formula II: cyclodextrin (molar ratio) | - | 1:37* | | |
| | | | | |
| Concentration of γ-cyclodextrin (% by mass) | 0.1 | 1 | 5 | |
| Solubility of compound of formula II (mg/mL) | 0.05* | 0.17 | 0.12 | |
| Compound of formula II: cyclodextrin (molar ratio) | 1:6* | 1:19 | 0.13958 | |
| | | | | |
| Concentration of hydroxypropyl-β-cyclodextrin (% by mass) | 0.25 | 5 | 10 | |
| Solubility of compound of formula II (mg/mL) | 0.10* | 2.0* | 3.0* | |
| Compound of formula II: cyclodextrin (molar ratio) | 1:7* | 1:7* | 1:9* | |

| | | | | |
|---|---|---|---|---|
| * : Actual solubility is assumed to be much higher because the compound of formula II was completely dissolved. | | | | |

Figure 1 illustrates the relation between the concentration of α-cyclodextrin and the solubility of the compound of formula II. The higher the concentration of α-cyclodextrin in the aqueous solution is, the higher the solubility of the compound of formula II is. When the concentration of α-cyclodextrin in the aqueous solution is 12% by mass, a solubility of the compound of formula II is 1.52 mg/mL. Figure 2 illustrates the ratios of the compound of formula II and α-cyclodextrin in Figure 1 in molar ratio. Considering the solubility of α-cyclodextrin in water is approximately 14% by mass, the lower limitation of a formulation ratio of the compound of formula II and α-cyclodextrin was set to 1:35.

Table 7 shows the solubilities of the compound of formula II in various cyclodextrin aqueous solutions. For β-cyclodextrin, a formulation ratio of the compound of formula II:β-cyclodextrin = 1:37 was possible at least in aqueous solution. However, the complex can also be prepared by ball milling as described in Example 6. Therefore, the minimum formulation ratio was 1:9 by mass, that is, approximately 1:3 by molar ratio. For γ-cyclodextrin, it was proved that when the concentration of γ-cyclodextrin in the aqueous solution became higher, the solubility of the compound of formula II became lower. Therefore, the lower limit of the formulation ratio was set to approximately 1:6. In the case of hydroxypropyl-β-cyclodextrin, since the compound of formula II was dissolved more than expected, the solubility could not be determined. However, a formulation ratio of at least 1:7 was possible.

Solubilities of α-cyclodextrin, β-cyclodextrin, γ-cyclodextrin and hydroxypropyl-β-cyclodextrin in water at 25°C are approximately 14%, approximately 2%, approximately 23% and approximately 22%, respectively.

### Industrial Applicability

According to the present invention, it is possible to provide an external preparation having excellent storage stability and content uniformity even in low concentration of the compound of formula I. Therefore, it is expected to provide an external preparation having excellent therapeutic effect on pruritic symptoms such as itching caused by atopic dermatitis.

### Brief Description of the Drawings

Figure 1 is a graph showing the relation between the concentration of α-cyclodextrin and the solubility of the compound of formula II in aqueous solutions at 25°C.
Figure 2 is a graph showing the relation between the concentration of α-cyclodextrin and the molar ratio of the compound of formula II and α-cyclodextrin in aqueous solutions at 25°C.

## Claims

1. An external preparation comprising a complex containing:
any one of a prostaglandin derivative, a pharmaceutically acceptable salt thereof and a hydrate thereof; and
a cyclodextrin,
the prostaglandin derivative being represented by the following formula (I): 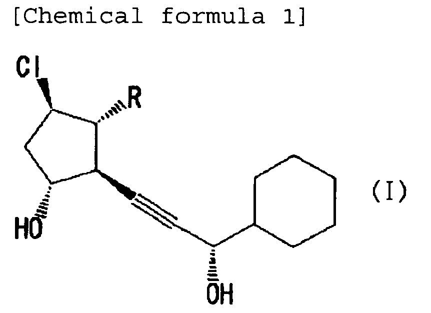 (where R represents a group represented by the formula: -(CH₂)₄-S-CH₂-CO₂H,- -(CH₂)₄-S-CH₂-CO₂CH₃, -(CH₂)₄-C=C-CO₂H, -CH₂-S- (CH₂)₂-S-CH₂-CO₂H, or -CH₂-S-(CH₂)₄-CO₂H).

2. An external preparation containing an active ingredient dispersed therein, wherein a complex containing: any one of a prostaglandin derivative represented by the formula (I), a pharmaceutically acceptable salt thereof, and a hydrate thereof; and a cyclodextrin is dispersed in an oleaginous base.

3. The external preparation according to claim 2, wherein the cyclodextrin is α-cyclodextrin, and a content of α-cyclodextrin is 35 to 216400 moles per mole of the one of the prostaglandin derivative represented by the formula (I), the pharmaceutically acceptable salt thereof and the hydrate thereof.

4. The external preparation according to claim 2, wherein the cyclodextrin is β-cyclodextrin, and a content of β-cyclodextrin is 3 to 185500 moles per mole of the one of the prostaglandin derivative represented by the formula (I), the pharmaceutically acceptable salt thereof and the hydrate thereof.

5. The external preparation according to claim 2, wherein the cyclodextrin is γ-cyclodextrin, and a content of γ-cyclodextrin is 6 to 162300 moles per mole of the one of the prostaglandin derivative represented by the formula (I), the pharmaceutically acceptable salt thereof and the hydrate thereof.

6. The external preparation according to claim 2, wherein the cyclodextrin is hydroxypropyl-β-cyclodextrin being one of β-cyclodextrin derivatives, and a content of hydroxypropyl-β-cyclodextrin is 7 to 139700 moles per mole of the one of the prostaglandin derivative represented by the formula (I), the pharmaceutically acceptable salt thereof and the hydrate thereof.

7. The external preparation according to any one of claims 2 to 6, wherein the external preparation is an ointment preparation.

8. An external preparation containing an active ingredient dissolved therein, wherein a complex comprising: any one of a prostaglandin derivative represented by the formula (I), a pharmaceutically acceptable salt thereof and a hydrate thereof; and a cyclodextrin is dissolved in an aqueous base.

9. The external preparation according to claim 8, wherein the cyclodextrin is α-cyclodextrin, and a content of α-cyclodextrin is 35 to 60600 moles per mole of the one of the prostaglandin derivative represented by the formula (I), the pharmaceutically acceptable salt thereof and the hydrate thereof.

10. The external preparation according to claim 8, wherein the cyclodextrin is β-cyclodextrin, and a content of β-cyclodextrin is 37 to 7400 moles per mole of the one of the prostaglandin derivative represented by the formula (I), the pharmaceutically acceptable salt thereof and the hydrate thereof.

11. The external preparation according to claim 8, wherein the cyclodextrin is γ-cyclodextrin, and a content of γ-cyclodextrin is 6 to 74600 moles per mole of the one of the prostaglandin derivative represented by the formula (I), the pharmaceutically acceptable salt thereof and the hydrate thereof.

12. The external preparation according to claim 8, wherein the cyclodextrin is hydroxypropyl-β-cyclodextrin being one of β-cyclodextrin derivatives, and a content of hydroxypropyl-β-cyclodextrin is 7 to 61400 moles per mole of the one of the prostaglandin derivative represented by the formula (I), the pharmaceutically acceptable salt thereof and the hydrate thereof.

13. The external preparation according to any one of claims 8 to 12, wherein the external preparation is a lotion preparation.
